(19) Europäisches Patentamt
European Patent Office
Office européen des brevets

(11) **EP 3 183 033 B1**

(12) **EUROPÄISCHE PATENTSCHRIFT**

(45) Veröffentlichungstag und Bekanntmachung des Hinweises auf die Patenterteilung:
**29.08.2018 Patentblatt 2018/35**

(21) Anmeldenummer: **15801158.5**

(22) Anmeldetag: **26.11.2015**

(51) Int Cl.:
**A61N 1/36** $^{(2006.01)}$

(86) Internationale Anmeldenummer:
**PCT/EP2015/077802**

(87) Internationale Veröffentlichungsnummer:
**WO 2016/083520 (02.06.2016 Gazette 2016/22)**

(54) **VORRICHTUNG ZUR EFFEKTIVEN NICHT-INVASIVEN NEUROSTIMULATION MITTELS VARIIERENDER REIZSEQUENZEN**

DEVICE FOR EFFECTIVE NON-INVASIVE NEUROSTIMULATION BY MEANS OF VARYING STIMULUS SEQUENCES

DISPOSITIF DE NEUROSTIMULATION NON-INVASIVE EFFICACE À L'AIDE DE SÉQUENCES D'EXCITATION VARIABLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.11.2014 DE 102014117427**

(43) Veröffentlichungstag der Anmeldung:
**28.06.2017 Patentblatt 2017/26**

(73) Patentinhaber: **Forschungszentrum Jülich GmbH 52425 Jülich (DE)**

(72) Erfinder:
• **TASS, Peter Alexander 83684 Tegernsee (DE)**
• **ZEITLER, Magteld 6581 JL Malden (NL)**

(74) Vertreter: **Müller-Boré & Partner Patentanwälte PartG mbB Friedenheimer Brücke 21 80639 München (DE)**

(56) Entgegenhaltungen:
**WO-A2-2010/112023         DE-A1-102010 000 390 DE-A1-102012 002 436**

**Beschreibung**

Die Erfindung betrifft eine Vorrichtung zur effektiven nicht-invasiven Neurostimulation mittels variierender Reizsequenzen.

Bei Patienten mit neurologischen oder psychiatrischen Erkrankungen, z. B. Morbus Parkinson, essentiellem Tremor, Dystonie, Funktionsstörungen nach Schlaganfall, Migräne, Zwangserkrankungen, Epilepsie, Tinnitus, Schizophrenie, Depression, Borderline Persönlichkeitsstörung sowie Reizdarmsyndrom, sind Nervenzellverbände in umschriebenen Bereichen des Gehirns krankhaft synchroner Weise aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität im Thalamus und in den Basalganglien die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten. Beim chronisch subjektivem Tinnitus findet sich krankhafte synchrone Aktivität in einem Netzwerk von auditorischen sowie nicht-auditorischen Hirnarealen.

Bei Patienten mit Hirnerkrankungen und Rückenmarkserkrankungen, welche durch übermäßig synchronisierte neuronale Aktivität gekennzeichnet sind, werden nicht-invasiv bestimmte raum-zeitliche Reizmuster, insbesondere die "Coordinated Reset"-Stimulation (CR-Stimulation) appliziert, um eine dauerhafte Linderung zu erzielen. Die nicht-invasive CR-Stimulation kann mittels verschiedener Stimulationsmodi realisiert werden:

(i) durch sensorische Reizung, d. h. durch physiologische Reizung von Rezeptoren, wie z. B. akustische Reizung des Innenohrs, visuelle Reizung der Retina oder mechanische (z. B. vibrotaktile) oder thermische Reizung von Haut-, Unterhaut-, Muskel- und Sehnenrezeptoren;

(ii) durch Reizung peripherer Nerven (und zugehöriger Rezeptoren) z. B. mittels elektrischem Strom (z. B. transkutane Elektrostimulation), mittels Magnetfeldern (transdermale Magnetstimulation) oder mittels Ultraschall; und

(iii) durch Reizung des Gehirns oder Rückenmarks, z. B. mittels elektrischem Strom (z. B. externe kraniale bzw. transkranielle Neurostimulation), mittels Magnetfeldern (z. B. transkranielle Magnetstimulation) oder mittels Ultraschall.

Zur Behandlung des chronisch subjektiven tonalen bzw. engbandigen Tinnitus wird die akustische CR-Stimulation verwandt. Hierzu werden Therapietöne an den dominanten Tinnituston angepasst und im Sinne der CR-Stimulation appliziert, um eine lang anhaltende, das Ausschalten der Stimulation deutlich überdauernde Desynchronisation der krankhaft synchronen Aktivität bzw. sogar eine anhaltende Desynchronisation derselben zu erzielen. Die akustische CR-Stimulation zur Behandlung des Tinnitus bewirkt eine signifikante und deutlich ausgeprägte Abnahme der Symptomatik (vgl. P. A. Tass, I. Adamchic, H.-J. Freund, T. von Stackelberg, C. Hauptmann: Counteracting tinnitus by acoustic coordinated reset neuromodulation. Restorative Neurology and Neuroscience 30, 137-159 (2012)), eine signifikante Abnahme der krankhaften neuronalen Synchronisation in einem Netzwerk von auditorischen und nicht-auditorischen Hirnarealen (vgl. P. A. Tass, I. Adamchic, H.-J. Freund, T. von Stackelberg, C.Hauptmann: Counteracting tinnitus by acoustic coordinated reset neuromodulation. Restorative Neurology and Neuroscience 30, 137-159 (2012); I. Adamchic, T. Toth, C. Hauptmann, P. A. Tass: Reversing pathologically increased EEG power by acoustic CR neuromodulation. Human Brain Mapping 35, 2099-2118 (2014)), eine signifikante Abnahme der krankhaften Interaktionen zwischen unterschiedlichen Hirnarealen im selben (vgl. A. N. Silchenko, I. Adamchic, C. Hauptmann, P. A. Tass: Impact of acoustic coordinated reset neuromodulation on effective connectivity in a neural network of phantom sound. Neuroimage 77, 133-147 (2013)) sowie in unterschiedlichen (vgl. I. Adamchic, B. Langguth, C. Hauptmann, P. A. Tass: Abnormal brain activity and cross-frequency coupling in the tinnitus network. Frontiers in Neuroscience 8, 284 (2014)) Frequenzbereichen.

In analoger Weise lässt sich die Parkinsonsche Erkrankung mittels vibrotaktiler CR-Stimulation behandeln. Weitere Indikationen stellen z. B. Epilepsien, Funktionsstörungen nach Schlaganfall, chronische Schmerzsyndrome (mittels vibrotaktiler und/oder thermischer CR-Stimulation), Migräne (z. B. mittels visueller CR-Stimulation) dar. Des Weiteren lassen sich diese Erkrankungen mit transkranieller Magnetstimulation oder direkter elektrischer Stimulation des Gehirns oder direkter Hirnstimulation mittels Ultraschall behandeln.

Alle drei oben genannten Stimulationsmodalitäten (i) bis (iii) weisen die drei im Folgenden genannten wesentlichen Nachteile auf:

a) Die Stimulationswirkung kann von Anwendung zu Anwendung, d. h. von Stimulationsepoche zu Stimulationsepoche, relevant variieren. Mit anderen Worten ist die Stimulationswirkung in relevantem Ausmaß von den Anfangsbedingungen des Organismus bzw. Nervensystems abhängig, bei denen die Stimulation gestartet wird. Wird z. B.

bei der einen Stimulationsepoche ein sehr guter Effekt erzielt, so ist dieser bei einer nächsten Stimulationsepoche eher unbefriedigend.

b) Bei der bisherigen Form der CR-Stimulation hängt der Stimulationserfolg zu stark von der Reizintensität ab. Variationen der Reizintensität sind bei der nicht-invasiven CR-Stimulation typischerweise nicht zu vermeiden. Z. B. passen die Patienten bei der akustischen CR-Stimulation zur Behandlung des Tinnitus die Intensität der CR-Töne an die Lautstärke der Umgebungsgeräusche an. Bei der vibrotaktilen CR-Stimulation kann z. B. der Anpressdruck und damit die Reizintensität von der Unterlage, auf der die Patienten die stimulierte Extremität samt vibrotaktiler Aktoren lagern, abhängen. Helligkeitsschwankungen der Umgebung führen zu unterschiedlichen Reizstärken bei der optischen Stimulation (z. B. mittels Transmissionsbrille). Bei der elektrischen Reizung der Haut hängt die Reizstärke von der Leitfähigkeit der Haut und damit z. B. vom Schwitzen und allgemein vom vegetativen Zustand und Gesamt- bzw. Gesundheitszustand des Patienten ab.

c) Die Reizstärke ist ganz generell in Relation zu charakteristischen Kenngrößen des zu stimulierenden Systems, also des Körpers bzw. Nervensystems zu sehen. Da diese Kenngrößen (z. B. bestimmte Ionenkonzentrationen, Flüssigkeitsvolumina, Hormonkonzentrationen etc.) schwanken und z. B. ausgeprägten tageszeitlichen Schwankungen unterworfen sind, sollte eine optimale Reizstärke entweder entsprechend nachgeregelt werden, oder es sollte ein Stimulationsverfahren verwandt werden, dessen Stimulationseffekte möglichst unabhängig von diesen Schwankungen sind.

Zusammengefasst ist die Wirkung der bisher verwandten CR-Stimulation nicht hinreichend robust gegenüber Schwankungen der Reizintensität sowie gegenüber charakteristischen Kenngrößen des zu stimulierenden Organismus bzw. Nervensystems (zu Beginn der Stimulation sowie im Verlauf der Stimulation) und insbesondere schwankt die Wirkung der CR-Stimulation von Stimulationsepoche zu Stimulationsepoche zu stark, d. h., es gibt zu viele Stimulationsepochen mit geringem Effekt.

Das Dokument DE-A-10 2010 000390 offenbart den nächstliegenden Stand der Technik.

Der Erfindung liegt die Aufgabe zugrunde, eine Vorrichtung anzugeben, die es ermöglicht, über einen weiten Intensitätsbereich verbesserte und insbesondere lang anhaltende therapeutische Effekte zu erzielen.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1 eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;

Fig. 2A eine schematische Darstellung einer CR-Stimulation mit schnell variierenden Reizsequenzen;

Fig. 2B eine schematische Darstellung einer CR-Stimulation mit langsam variierenden Reizsequenzen;

Fig. 3 eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;

Fig. 4 eine schematische Darstellung einer Vorrichtung zur akustischen Stimulation von Neuronen mit einer krankhaft synchronen und oszillatorischen neuronalen Aktivität;

Fig. 5 eine schematische Darstellung einer Vorrichtung zur visuellen Stimulation von Neuronen mit einer krankhaft synchronen und oszillatorischen neuronalen Aktivität;

Fig. 6 eine schematische Darstellung einer Vorrichtung zur taktilen, vibratorischen, thermischen, transkutanen elektrischen und/oder transkutanen magnetischen Stimulation und/oder Ultraschall-Stimulation von Neuronen mit einer krankhaft synchronen und oszillatorischen neuronalen Aktivität; und

Fig. 7 bis 9 Diagramme mit Simulationsergebnissen für schnell und langsam variierende CR-Stimulationen.

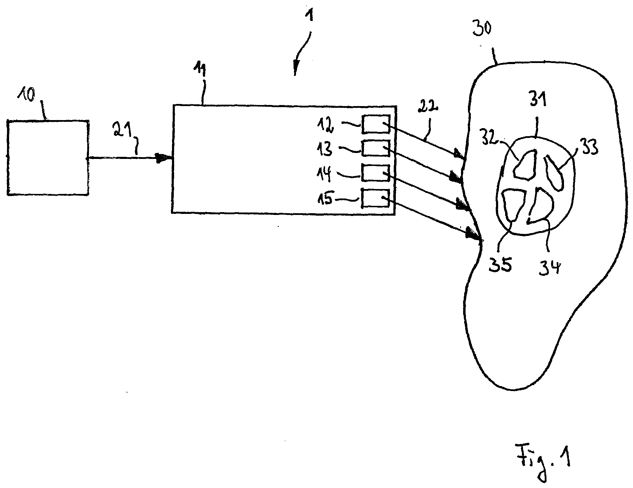

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10 und einer Stimulationseinheit 11, die über eine Mehrzahl von Stimulationskanälen Neuronen im Gehirn und/oder Rückenmark 30 eines Patienten stimuliert. Jeder Stimulationskanal ermöglicht die Stimulation eines anderen Zielgebiets im Gehirn und/oder Rückenmark 30 des Patienten, wobei die den Stimulationskanälen zugeordneten Zielgebiete nicht notwendigerweise disjunkt, d. h. vollständig voneinander getrennt sein müssen, sondern einander auch überlappen können. In Fig. 1 ist beispielhaft die Stimulation über vier Stimulationskanäle 12, 13, 14 und 15 dargestellt. Es kann selbstverständlich aber auch über eine andere Zahl von Stimulationskanälen stimuliert werden.

Während des Betriebs der Vorrichtung 1 führt die Steuereinheit 10 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 erzeugt anhand der Steuersignale 21 Reize 22 in den Stimulationskanälen 12 bis 15, die dem Patienten verabreicht werden. Die Reize 22 können sensorische Reize, z. B. akustische, visuelle, taktile, vibratorische, thermische, olfaktorische, gustatorische, transkutane elektrische, transkutane magnetische, transkranielle elektrische und/oder transkranielle magnetische Reize und/oder Ultraschall-Reize, sein. Insbesondere taktile und vibratorische Reize 22 werden auch gemeinsam appliziert und werden dann als vibrotaktile Reize 22 bezeichnet. Die Reize 22 können vom Patienten insbesondere bewusst wahrnehmbar sein. Die Reize 22 sind dazu ausgelegt, bei einer Verabreichung an den Patienten über die Stimulationskanäle 12 bis 15 die krankhaft synchrone und oszillatorische neuronale Aktivität zu unterdrücken und insbesondere die Neuronen mit der krankhaft synchronen und oszillatorischen Aktivität zu desynchronisieren.

Die Stimulationseinheit 11 und insbesondere auch die Steuer- und Analyseeinheit 10 sind nicht-invasive Einheiten, d. h., während des Betriebs der Vorrichtung 1 befinden sie sich außerhalb des Körpers des Patienten und werden nicht operativ in den Körper des Patienten implantiert.

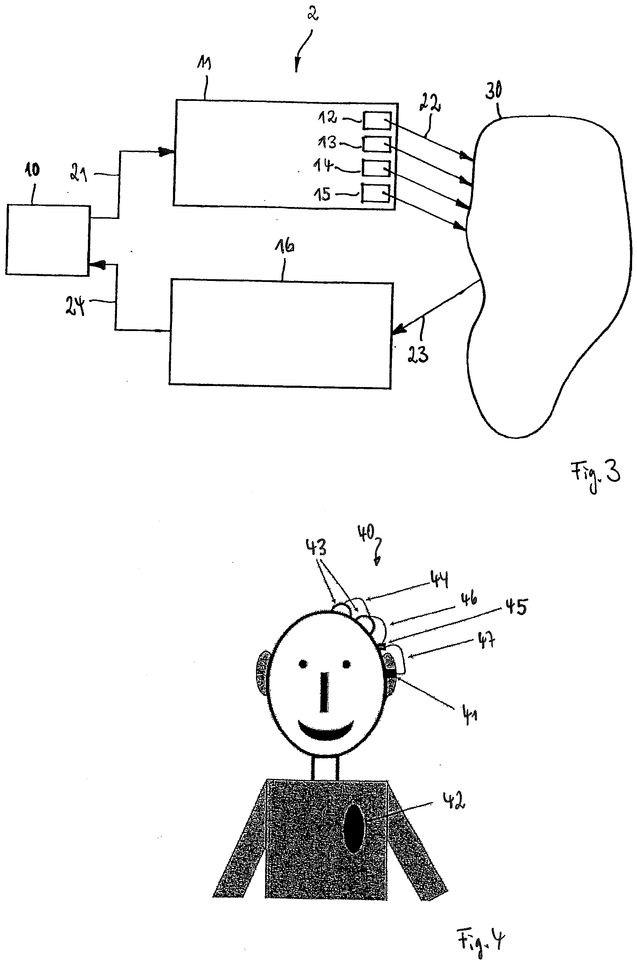

Die Vorrichtung 1 und die weiter unten, im Zusammenhang mit Fig. 3 beschriebene Vorrichtung 2 können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

In Fig. 1 ist die Vorrichtung 1 während einer CR-Stimulation dargestellt. Im Gehirn und/oder Rückenmark 30 des Patienten weist mindestens eine Neuronenpopulation 31 eine wie vorstehend beschrieben krankhaft synchrone und oszillatorische neuronale Aktivität auf. Die Stimulationseinheit 11 erzeugt sensorische Reize 22, die von dem Patienten aufgenommen werden und über das Nervensystem an die krankhaft aktive Neuronenpopulation 31 im Gehirn und/oder Rückenmark 30 weitergeleitet werden. Die Reize 22 sind so ausgestaltet, dass die zeitversetzte (oder phasenverschobene) Stimulation über mindestens zwei Stimulationskanäle eine Desynchronisation der krankhaft synchronen Aktivität der Neuronenpopulation 31 bewirkt. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize 22 bewirken in der Neuronenpopulation 30 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 31 mittels einer gezielten Stimulation kontrolliert. Da die krankhafte Neuronenpopulation 31 über die Stimulationskanäle 12 bis 15 an unterschiedlichen Stellen stimuliert wird, können die Phasen der neuronalen Aktivität der in Fig. 1 dargestellten Subpopulationen 32 bis 35 der krankhaften Neuronenpopulation 31 zu unterschiedlichen Zeitpunkten zurückgesetzt werden, indem die Reize 22 über die Stimulationskanäle 12 bis 15 zeitversetzt (oder phasenverschoben) appliziert werden. Im Ergebnis wird dadurch die krankhafte Neuronenpopulation 31, deren Neuronen zuvor synchron und mit gleicher Frequenz und Phase aktiv waren, in mehrere Subpopulationen mit unterschiedlichen Phasen aufgespalten. Beispielsweise wird über den Stimulationskanal 12 die Subpopulation 32 stimuliert, über den Stimulationskanal 13 wird die Subpopulation 33 stimuliert, über den Stimulationskanal 14 wird die Subpopulation 34 stimuliert und über den Stimulationskanal 15 wird die Subpopulation 35 stimuliert. Innerhalb jeder der Subpopulationen 32 bis 35 sind die Neuronen nach dem Zurücksetzen der Phase weiterhin synchron und feuern auch weiterhin mit derselben pathologischen Frequenz, aber jede der Subpopulationen 32 bis 35 weist bezüglich ihrer neuronalen Aktivität die Phase auf, die ihr durch den im jeweiligen Stimulationskanal 12 bis 15 generierten Reiz 22 aufgezwungen wurde. Dies bedeutet, dass die neuronalen Aktivitäten der einzelnen Subpopulationen 32 bis 35 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 31 nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 31 in Subpopulationen 32 bis 35 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

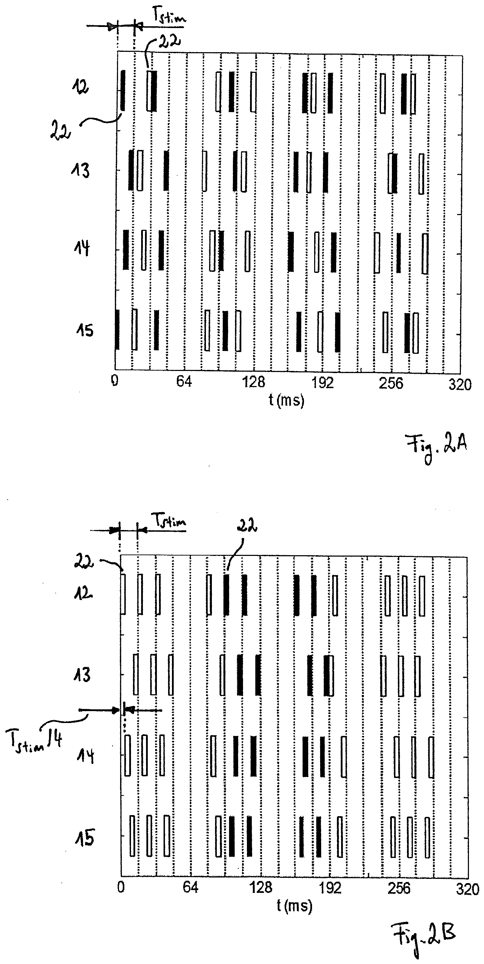

Fig. 2A zeigt eine CR-Stimulation, bei der in vier Stimulationskanälen 12 bis 15 repetitiv Sequenzen von Reizen 22 erzeugt werden. In Fig. 2A sind untereinander die in den Stimulationskanälen 12 bis 15 erzeugten Reize 22 gegen die Zeit t aufgetragen. Die Sequenzen werden in einem vorgegebenen Zeitraster, das aus aufeinanderfolgenden Zyklen besteht, erzeugt. Die einzelnen Zyklen sind in Fig. 2A durch gestrichelte Linien voneinander abgegrenzt. Jeder Zyklus weist die Länge $T_{stim}$ auf. In jedem Zyklus, in dem eine Stimulation erfolgt, wird in den Stimulationskanälen 12 bis 15 zusammen genau eine Sequenz von Reizen 22 erzeugt und in jedem Stimulationskanal 12 bis 15 wird genau einen Reiz 22 erzeugt, d. h., jede Sequenz besteht in dem vorliegenden Beispiel aus einer Abfolge von vier zeitversetzten Reizen 22, die insbesondere in jeweils unterschiedlichen Stimulationskanälen 12 bis 15 generiert werden, wobei sich der Zeitversatz insbesondere auf die Anfangszeitpunkte der Reize 22 beziehen kann. Zu Beginn jedes Zyklus wird in dem vorliegenden Beispiel die Reihenfolge der Stimulationskanäle 12 bis 15, in denen die Reize 22 innerhalb der jeweiligen Sequenz erzeugt werden, variiert. Eine unterschiedliche Füllung der in Fig. 2A gezeigten Balken, welche die Reize 22 symbolisieren, zeigt eine Variation der Reihenfolge an. Beispielsweise werden die Reize 22 in den Stimulationskanälen 12 bis 15 in dem ersten in Fig. 2A gezeigten Zyklus in der Reihenfolge 15-12-14-13 erzeugt. Im zweiten Zyklus lautet die Reihenfolge 15-13-14-12 und im dritten Zyklus lautet die Reihenfolge 12-15-14-13.

Ferner werden bei der in Fig. 2A gezeigten beispielhaften Stimulationsform stets in drei aufeinanderfolgenden Zyklen Reize 22 appliziert und danach wird für zwei Zyklen eine Pause eingehalten, in der keine Reize 22 generiert werden. Dieses Muster wird periodisch wiederholt.

Fig. 2B zeigt eine Weiterentwicklung der CR-Stimulation von Fig. 2A. Der wesentliche Unterschied zur Stimulation nach Fig. 2A besteht darin, dass bei der in Fig. 2B dargestellten CR-Stimulation die Sequenzen nur sehr langsam variiert werden. Insbesondere ist vorgesehen, dass die Reihenfolge der Stimulationskanäle 12 bis 15, in denen die Reize 22 innerhalb einer Sequenz erzeugt werden, für mindestens 20 nacheinander generierte Sequenzen konstant gehalten und erst danach variiert wird. Eine CR-Stimulation mit derart langsam variierenden Sequenzen ist gegenüber der in Fig. 2A gezeigten CR-Stimulation erheblich überlegen, da ihr gewünschter, d. h. therapeutischer Stimulationseffekt (i) stärker ausgeprägt ist, (ii) von Stimulationsepoche zu Stimulationsepoche deutlich weniger variiert und (iii) deutlich robuster gegenüber Schwankungen der Reizintensität, gegenüber Schwankungen charakteristischer Kenngrößen des Körpers bzw. Nervensystems sowie insbesondere gegenüber Variationen der Anfangswerte ist.

Allgemein bekannt ist, dass beim Lernen die Wiederholung des zu lernenden Inhalts eine wichtige Rolle spielt. Die Erfindung nutzt den überraschenden Zusammenhang, dass auch beim Verlernen das Wiederholen von überaus großer Bedeutung ist. D. h., um krankhaft synchrone synaptische Vernetzungen und damit krankhaft synchrone neuronale Aktivität erheblich besser zu verlernen, sollten die Sequenzen der CR-Stimulation nur langsam variiert werden, so dass jede einzelne Sequenz häufig genug wiederholt wird.

Wie oben beschrieben kann vorgesehen sein, dass die Sequenzen für mindestens 20 nacheinander generierte Sequenzen gleich bleiben und erst danach geändert werden. Es ist weiterhin denkbar, die Wiederholung derselben Sequenz zu erhöhen und die Reihenfolge der Stimulationskanäle 12 bis 15, in denen die Reize 22 innerhalb einer Sequenz erzeugt werden, für mindestens 25 oder mindestens 30 nacheinander generierte Sequenzen konstant zu halten. Es sei an dieser Stelle noch darauf hingewiesen, dass in Fig. 2B aus Gründen der Veranschaulichung die Sequenzen bereits nach weniger als 20 nacheinander generierten Sequenzen variiert werden. Dies ist jedoch lediglich als eine vereinfachte Darstellung einer im Vergleich zu Fig. 2A langsamen Sequenzvariation zu verstehen.

Gemäß einer Ausgestaltung wird bei der in Fig. 2B gezeigten CR-Stimulation nur die Reihenfolge der Stimulationskanäle 12 bis 15, in denen die Reize 22 innerhalb einer Sequenz erzeugt werden, variiert. Alle übrigen Stimulationsparameter können während der CR-Stimulation konstant bleiben.

Die Variation der Sequenzen kann z. B. stochastisch oder deterministisch oder gemischt stochastisch-deterministisch erfolgen.

Genauso wie in Fig. 2A können auch bei der CR-Stimulation nach Fig. 2B Zyklen vorgesehen sein, in denen Stimulationspausen eingehalten werden. So können während n aufeinanderfolgenden Zyklen Reize 22 erzeugt werden und während der darauffolgenden m Zyklen keine Reize 22 erzeugt werden, die dazu ausgelegt sind, die krankhaft synchrone und oszillatorische neuronale Aktivität zu unterdrücken, wobei n und m nicht-negative ganze Zahlen sind. Es ist jedoch denkbar, dass andere Reize, die nicht dazu ausgelegt sind, die krankhaft synchrone und oszillatorische neuronale Aktivität zu unterdrücken, während der Stimulationspausen insbesondere mit der Stimulationseinheit 11 appliziert werden. Weiterhin kann vorgesehen sein, dass die Stimulationseinheit 11 während der Stimulationspausen keinerlei Reize erzeugt. Das Muster aus n Zyklen mit Stimulation und m Zyklen ohne Stimulation kann periodisch fortgesetzt werden.

Sofern vorgesehen ist, die Sequenzen nach einer vorgegebenen Anzahl i von nacheinander generierten Sequenzen zu variieren ($i \geq 20$), werden gemäß einer Ausgestaltung die Zyklen ohne Stimulation nicht mitgezählt, d. h., es findet bei dieser Ausgestaltung eine Variation der Reihenfolge der Stimulationskanäle 12 bis 15 erst dann statt, wenn tatsächlich in i Zyklen jeweils eine Sequenz von Reizen 22 erzeugt wurde. Die Anzahl i, nach der jeweils die Sequenz variiert wird, kann z. B. gemäß stochastischen oder deterministischen oder gemischt stochastisch-deterministischen Regeln bestimmt werden.

Weiterhin kann die Variation der Sequenzen mit einem konstanten Rhythmus erfolgen, d. h., eine Variation findet beispielsweise stets nach i Zyklen statt.

Über jeden der vier Stimulationskanäle 12 bis 15 wird eine jeweilige der in Fig. 1 dargestellten Subpopulationen 32 bis 34 der krankhaften Neuronenpopulation 31 stimuliert. Während der mindestens 20 Zyklen, in denen die Sequenzen konstant sind, wird über jeden der vier Stimulationskanäle 12 bis 15 der Reiz 22 periodisch mit der Periode $T_{stim}$ appliziert. Die Reize 22 bewirken eine Phasenrücksetzung der neuronalen Aktivität der jeweils stimulierten Subpopulation. Ferner beträgt die zeitliche Verzögerung zwischen innerhalb einer Sequenz zeitlich direkt aufeinanderfolgenden, in unterschiedlichen Stimulationskanälen erzeugten Reizen 22 $T_{stim}/4$, da in dem vorliegenden Ausführungsbeispiel in vier Stimulationskanälen 12 bis 15 die Reize 22 erzeugt werden. Für den allgemeinen Fall von P für die Stimulation verwendeten Stimulationskanälen würde die zeitliche Verzögerung zwischen innerhalb einer Sequenz zeitlich direkt aufeinanderfolgenden, in unterschiedlichen Stimulationskanälen erzeugten Reizen 22 $T_{stim}/P$ betragen (von diesem Wert kann auch um z. B. bis zu $\pm$ 5%, $\pm$ 10% oder $\pm$ 20% abgewichen werden). Die zeitliche Verzögerung $T_{stim}/P$ kann sich auf die Anfangszeitpunkte der Reize 22 beziehen. Die in unterschiedlichen Stimulationskanälen erzeugten Reize 22 können bis auf die unterschiedlichen Startzeitpunkte identisch sein.

Die Periode $T_{stim}$, die zum einen die Dauer eines Zyklus und zum anderen die Periode angibt, mit der gleich bleibende Sequenzen sowie die in einem jeweiligen Stimulationskanal 12 bis 15 generierten Reize 22 wiederholt werden, kann nahe bei der mittleren Periode der pathologischen Oszillation der Neuronenpopulation 31 mit der krankhaft synchronen und oszillatorischen neuronalen Aktivität liegen bzw. um bis zu $\pm$ 5%, $\pm$ 10% oder $\pm$ 20% von der mittleren Periode abweichen. Typischerweise liegt die Frequenz $f_{stim} = 1/T_{stim}$ im Bereich von 1 bis 30 Hz. Die Periode der pathologischen Oszillation der zu stimulierenden Neuronenpopulation 31 kann beispielsweise mittels der unten beschriebenen Messeinheit 16, insbesondere mittels EEG, gemessen werden. Es ist aber auch möglich, für die Periode der pathologischen Oszillation Literatur- oder Erfahrungswerte, die sich auf die jeweilige, zu behandelnde Krankheit beziehen, zu verwenden.

Die phasenrücksetzenden Reize 22 können beispielsweise Einzelreize oder auch zusammengesetzte Reize sein. Beispielsweise kann jeder Reiz 22 aus einem Pulszug mit 1 bis 100, insbesondere 2 bis 10 Einzelpulsen bestehen. Innerhalb eines Pulszugs werden die Einzelpulse ohne Unterbrechung mit einer Frequenz im Bereich von 50 bis 500 Hz, insbesondere im Bereich von 100 bis 150 Hz, wiederholt. Je nach Stimulationsart kann es sich um einen akustischen, visuellen, taktilen, vibratorischen (insbesondere vibrotaktilen), thermischen, olfaktorischen, gustatorischen, transkutanen elektrischen, transkutanen magnetischen, transkraniellen elektrischen und/oder transkraniellen magnetischen Pulszug und/oder einen Ultraschall-Pulszug handeln.

Grundsätzlich kann es möglich sein, mittels der Stimulationseinheit 11 über eine beliebige Anzahl L von Stimulationskanälen zu stimulieren (L ≥ 2), jedoch müssen bei einer Stimulation nicht zwingend in allen L Stimulationskanälen Reize 22 erzeugt werden, es können beispielsweise auch in nur einer Auswahl von P der L Stimulationskanäle die Reize 22 erzeugt werden (2 ≤ P ≤ L). Bei P Stimulationskanälen ergeben sich P! mögliche unterschiedliche Sequenzen, wobei bei jeder dieser Sequenzen in jedem der P Stimulationskanäle genau ein Reiz 22 erzeugt wird. Es ist denkbar, alle P! möglichen Sequenzen für die Stimulation heranzuziehen oder auch aus der Menge der P! möglichen Sequenzen eine Untermenge für die Stimulation auszuwählen. Diese Untermenge kann auch in der Zeit gemäß stochastisch oder deterministisch oder gemischt stochastisch-deterministisch Regeln variieren. Die Abfolge der Sequenzen kann zufällig sein oder vor Beginn oder auch während der Stimulation festgelegt werden.

Die in Fig. 1 dargestellte Vorrichtung 1 zur Stimulation von Neuronen mit einer krankhaft synchronen und oszillatorischen neuronalen Aktivität führt eine sogenannte "open loop"-Stimulation durch, d. h., eine Stimulation ohne Sensoren, die zur Rückmeldung und/oder Steuerung der Stimulation verwendet werden.

Fig. 3 zeigt schematisch eine Vorrichtung 2 zur Stimulation von Neuronen mit einer krankhaft synchronen und oszillatorischen neuronalen Aktivität, mit der sich eine "closed loop"-Stimulation durchführen lässt. Die Vorrichtung 2 ist eine Weiterbildung der in Fig. 1 dargestellten Vorrichtung 1 und enthält genauso wie die Vorrichtung 1 eine Steuereinheit 10 und eine nicht-invasive Stimulationseinheit 11, welche die gleichen Funktionen wie die oben beschriebenen Steuer- und Stimulationseinheiten 10, 11 der Vorrichtung 1 aufweisen.

Darüber hinaus umfasst die Vorrichtung 2 eine Messeinheit 16. Der durch die Reize 22 erzielte Stimulationseffekt wird mit Hilfe der Messeinheit 16 überwacht. Die Messeinheit 16 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 16 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Mittels der Messeinheit 16 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind.

Die Messeinheit 16 enthält ein oder mehrere Sensoren, die es insbesondere ermöglichen, eine Ab- bzw. Zunahme der Amplitude der pathologischen oszillatorischen Aktivität nachzuweisen.

Als Sensoren können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden.

Alternativ, aber weniger bevorzugt können die Sensoren in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen.

Die Steuereinheit 10 verarbeitet die Signale 24, z. B. können die Signale 24 verstärkt und/oder gefiltert werden, und analysiert die verarbeiteten Signale 24. Die Steuereinheit 10 überprüft anhand der in Reaktion auf die Applikation der Reize 22 aufgenommenen Messsignale 23 den Stimulationserfolg.

Gemäß einer Ausgestaltung werden die von der Stimulationseinheit 11 erzeugten Sequenzen mit konstantem Rhythmus variiert, d. h., die Reihenfolge der Stimulationskanäle 12 bis 15, in denen die Reize 22 pro Sequenz erzeugt werden, wird für eine vorgegebene Zahl von Zyklen (mindestens 20) konstant gehalten und danach wird die Reihenfolge variiert. Anschließend wird die Reihenfolge für die vorgegebene Zahl von Zyklen wieder konstant gehalten und danach variiert. Dieses Muster wird entsprechend fortgesetzt. Bei dieser Ausgestaltung bleibt der Rhythmus, mit dem die Sequenzen variiert werden, konstant und wird insbesondere nicht an die von der Steuereinheit 10 verarbeiteten Messsignale 23 angepasst, jedoch können bei Bedarf andere Stimulationsparameter, wie z. B. die Amplitude der Reize 22, in Abhängigkeit von den Messsignalen 23 eingestellt werden.

Die vorstehende Ausgestaltung kann weitergebildet werden, indem der Rhythmus der Sequenzen in Abhängigkeit von den verarbeiteten Messsignalen 23 eingestellt wird. Die Steuereinheit 10 überprüft anhand der in Reaktion auf die Applikation der Reize 22 aufgenommenen Messsignale 23 den Stimulationserfolg und stellt die Stimulationsparameter, insbesondere den Rhythmus, mit der die Stimulationssequenzen variiert werden, in Abhängigkeit vom Stimulationserfolg ein.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 16, z. B. EEG-Elektroden, gemessen, reicht erfahrungsgemäß die Absenkung der Synchronisation um z. B. mindestens 20 % im Vergleich zur Situation ohne Stimulation, um einen ausreichenden Stimulationserfolg festzustellen. Gemäß einer Ausgestaltung kann ein nicht ausreichender Stimulationserfolg festgestellt werden, wenn sich die krankhafte neuronale Synchronisation durch die Applikation der Reize 22 nicht um mindestens einen vorgegebenen Wert verringert. Falls Symptome des Patienten zur Ermittlung des Stimulationserfolgs herangezogen werden, hängt es von der Art der verwandten klinischen Parameter ab, welche Abnahme als klinisch relevante Besserung anzusehen ist. Derartige Abnahmewerte (z. B. im Sinne der sog. minimalen klinisch wahrnehmbaren Verbesserung) sind dem Fachmann bekannt.

Wenn die CR-Stimulation gemäß Schwellwertvergleich nicht hinreichend effektiv ist, d. h., ein krankheitsspezifischer Marker im Vergleich zum Ausgleichszustand bzw. zu einem Start-/Ausgangswert durch die CR-Stimulation nicht um einen vorgegebenen Schwellwert abnimmt, wird die Anzahl der Wiederholungen derselben Sequenz verlängert. Ist die Stimulation hingegen gemäß Schwellwertkriterium erfolgreich, wird die Anzahl der Wiederholungen derselben Sequenz verkürzt. Im einfachsten Fall kann dies ein binäres Schalten zwischen zwei Werten der Anzahl der Wiederholungen derselben Sequenz sein: z. B. 25 Wiederholungen bei erfolgreicher Stimulation, hingegen z. B. 100 Wiederholungen bei nicht erfolgreicher Stimulation. Die bedarfsgesteuerte Anzahl der Wiederholungen derselben Sequenz kann aber auch in kleineren Schritten variiert/parametrisiert werden.

Die einzelnen Komponenten der Vorrichtungen 1 und 2, insbesondere die Steuereinheit 10, die Stimulationseinheit 11 und/oder die Messeinheit 16, können baulich voneinander getrennt sein. Die Vorrichtungen 1 und 2 können daher auch als Systeme aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

Fig. 4 zeigt schematisch eine Vorrichtung 40 zur nicht-invasiven akustischen Stimulation von Neuronen mit einer krankhaft synchronen und oszillatorischen neuronalen Aktivität gemäß einer Ausführungsform der Erfindung. Akustische Reize, insbesondere akustische CR-Reize, werden dem Patienten über Ohr- oder Kopfhörer 41 oder anders ausgestaltete Lautsprecher verabreicht, wobei ein Ohrhörer ein im Ohrkanal platzierter Lautsprecher ist. Die hierzu verwendeten Steuersignale werden von einer Steuereinheit 42 generiert. Nicht-invasiv fixierte EEG-Elektroden 43, die über ein Kabel 44 verbunden sind, dienen der "closed loop"-Stimulation. Die entsprechende Verrechnung wird in einem kleinen Bauteil 45, das vorzugsweise einen Messverstärker enthält und über Kabel 46, 47 mit den EEG-Elektroden 43 bzw. dem Ohr- oder Kopfhörer 41 verbunden ist, und/oder in der eigentlichen, die Batterie bzw. den Akku beherbergenden Steuereinheit 42 durchgeführt. Die Steuereinheit 42 und das Bauteil 45 sind in der in Fig. 4 dargestellten Ausführungsform telemetrisch miteinander verbunden; in diesem Fall enthält Bauteil 45 (oder ein mit ihm über Kabel verbundenes Bauteil) ebenso eine Batterie bzw. einen Akku. Alternativ können die Steuereinheit 42 und das Bauteil 45 auch über Kabel miteinander verbunden sein, so dass das Bauteil 45 über die Stromversorgung von der Steuereinheit 42 gespeist wird.

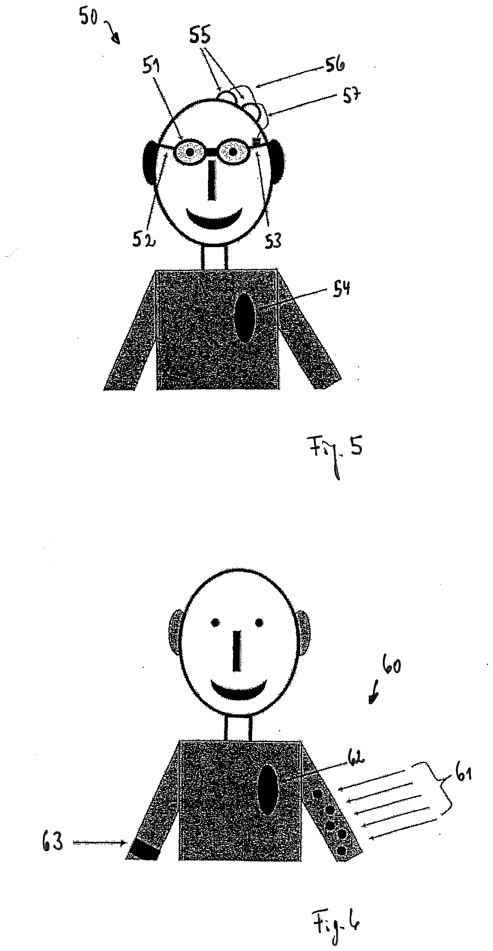

Fig. 5 zeigt schematisch eine Vorrichtung 50 zur nicht-invasiven visuellen Stimulation von Neuronen mit einer krankhaft synchronen und oszillatorischen neuronalen Aktivität gemäß einer Ausführungsform der Erfindung. Bei dieser Ausgestaltung trägt der Patient eine Stimulationsbrille 51, die z. B. über Bügel 52 am Kopf des Patienten befestigt ist. Ein Bauteil 53 enthält eine Verrechnungs- und Telemetrieeinheit. Letztere dient der Verbindung mit der eigentlichen, die Batterie bzw. den Akku beherbergenden Steuereinheit 54. Das Bauteil 53 und die Steuereinheit 54 sind telemetrisch miteinander verbunden; in diesem Fall enthält Bauteil 53 (oder ein mit ihm über Kabel verbundenes Bauteil) ebenso eine Batterie bzw. einen Akku. Alternativ können das Bauteil 53 und die Steuereinheit 54 auch über Kabel miteinander verbunden sein. Nicht-invasiv fixierte EEG-Elektroden 55 dienen der "closed loop"-Stimulation. Die EEG-Elektroden 55 sind über Kabel 56, 57 mit dem Bauteil 53 verbunden.

Den von der Stimulationsbrille 51 erzeugten visuellen Reizen kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die visuellen Reize können je nach Ausgestaltung als Leuchtstärkenmodulation natürlicher visueller Reize, z. B. mittels einer homogenen oder segmentierten Transmissionsbrille, bei der spannungsabhängig die Transmission geregelt werden kann, als zusätzlich zu einem natürlichen visuellen Reiz auftretender, modulierter visueller Reiz, z. B. mittels einer partiell durchsichtigen Lichtbrille, oder als künstlicher visueller Helligkeitsreiz, z. B. mittels einer undurchsichtigen Lichtbrille, verabreicht werden. Die Stimulationsbrille 51 ist vorzugsweise in unterschiedliche Segmente unterteilt, deren Leuchtstärke bzw. Transmission bzw. Helligkeit getrennt gesteuert werden kann, um verschiedene Stellen der Retina unabhängig voneinander stimulieren zu können.

Fig. 6 zeigt schematisch eine Vorrichtung 60 zur nicht-invasiven taktilen, vibratorischen, thermischen, transkutanen elektrischen und/oder transkutanen magnetischen Stimulation und/oder Ultraschall-Stimulation von Neuronen mit einer krankhaft synchronen und oszillatorischen neuronalen Aktivität gemäß einer Ausführungsform der Erfindung. Die Vorrichtung 60 umfasst eine Stimulationseinheit 61, eine die Stimulationseinheit 61 ansteuernde Steuereinheit 62 und ein Akzelerometer (Beschleunigungsmesser) 63 zum Aufnehmen von Messsignalen. Die Stimulationseinheit 61 und das Akzelerometer 63 können mit der Steuereinheit 62 telemetrisch oder über Kabel verbunden sein.

Die Stimulationseinheit 61 umfasst eine Mehrzahl von Stimulationselementen zur Erzeugung von taktilen, vibratorischen, thermischen, transkutanen elektrischen und/oder transkutanen magnetischen Reizen und/oder Ultraschall-Reizen. Die Stimulationselemente sind derart ausgestaltet, dass sie auf die Haut des Patienten aufgesetzt werden können. Je nach Erkrankung bzw. betroffenen Körperpartien werden die Stimulationselemente in einer geeigneten Anordnung auf der Haut des Patienten befestigt, beispielsweise am Arm, am Bein, an der Hand und/oder am Fuß des Patienten. Die Mehrzahl von Stimulationselementen ermöglicht es, unterschiedliche rezeptive Bereiche der Haut über die einzelnen Stimulationselemente zeitlich und räumlich koordiniert zu stimulieren.

Stimulationselemente zur Erzeugung von taktilen und/oder vibratorischen Reizen sind beispielsweise Vibrationsaktoren, die mit einer Frequenz im Bereich von 1 bis 300 Hz und insbesondere 1 bis 60 Hz und vorzugsweise 100 bis 300 Hz in die Haut des Patienten eindrücken und dadurch die gewünschten Reize erzeugen. Stimulationselemente zur Erzeugung thermischer Reize können beispielsweise Laser oder anders ausgestaltete Elemente zur Erzeugung von Wärme, insbesondere Wärmestrahlung, sein. Zur Erzeugung von transkutanen elektrischen Reizen werden typischerweise Elektroden auf der Haut des Patienten befestigt. Transkutane magnetische Reize können durch entsprechende Stimulationselemente zur Erzeugung magnetischer Reize, insbesondere stromdurchflossene Spulen, erzeugt werden. Ultraschall-Reize werden durch Stimulationselemente zur Erzeugung von Ultraschall-Wellen erzeugt.

Bei der Applikation akustischer oder visueller Reize werden diese über mindestens ein Ohr bzw. mindestens ein Auge des Patienten aufgenommen. Die taktilen, vibratorischen, thermischen, transkutanen elektrischen und/oder transkutanen magnetischen Reize und/oder Ultraschall-Reize können von in oder unter der Haut gelegenen Rezeptoren aufgenommen und an das Nervensystem weitergeleitet werden. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize wirken. Die vibratorischen Reize zielen vorwiegend auf die Tiefensensibilität ab. Die vibratorischen Reize können von in der Haut, den Muskeln, dem Subkutangewebe und/oder den Sehnen des Patienten gelegenen Rezeptoren aufgenommen werden. Als Rezeptoren für die vibratorischen Reize seien beispielhaft die Vater-Pacini-Körperchen genannt, die Vibrationsempfindungen und Beschleunigungen vermitteln. Die thermischen Reize werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer. Die transkutanen elektrischen und transkutanen magnetischen Reize sowie die Ultraschall-Reize wirken nicht spezifisch auf nur eine Gruppe von in oder unter der Haut gelegenen Rezeptoren und können darüber hinaus auch direkt Nervenfasern reizen.

Die gezielte Stimulation bestimmter Bereiche des Gehirns oder Rückenmarks wird durch die tonotope bzw. somatotope Zuordnung von Körperregionen zu diesen Bereichen ermöglicht. Beispielsweise werden akustische Reize im Innenohr in Nervenimpulse umgesetzt und über den Hörnerv zu dem auditorischen Cortex weitergeleitet. Durch die tonotope Anordnung des auditorischen Cortex wird bei der akustischen Stimulation des Innenohres mit einer bestimmten Frequenz ein bestimmter Teil des auditorischen Cortex aktiviert.

Bei der visuellen Stimulation werden unterschiedliche Stellen im Gesichtsfeld über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Folglich können mit den an unterschiedlichen räumlichen Orten applizierten Reizen jeweils unterschiedliche Neuronen stimuliert werden.

Aufgrund der somatotopen Gliederung der Nervenleitungsbahnen und zugehörigen Hirngebiete werden des Weiteren durch taktile, vibratorische, thermische, transkutane elektrische und/oder transkutane magnetische Reize und/oder Ultraschall-Reize, die an unterschiedlichen Stellen der Haut appliziert werden, unterschiedliche Neuronen stimuliert. Bei diesen Stimulationsformen können die Stimulationselemente beispielsweise am Fuß, Unterschenkel und Oberschenkel oder aber an der Hand, dem Unterarm und Oberarm des Patienten angebracht werden, um dadurch bestimmte Neuronen stimulieren zu können.

Bei der gustatorischen Reizung werden unterschiedliche Bereich der Zunge mit den entsprechenden Geschmacksqualitäten - süß, sauer, salzig, bitter und umami (japanisch für pikantes, würziges, bouillonartiges Aroma) - gereizt. Es ist aber auch möglich, die Zunge elektrisch zu reizen. In diesem Fall reizt man primär die Schleimhaut, welche im Homunculus (Repräsentation der Oberfläche des Menschen im sensomotorischen Cortex) eine erheblich große Repräsentation, d. h. ein erheblich großes zugeordnetes Areal, aktiviert. Aufgrund der somatotopen Gliederung der Nervenleitungsbahnen und zugehörigen Hirngebiete werden durch gustatorische Reize, die an unterschiedlichen Stellen der Zunge appliziert werden, unterschiedliche Neuronen stimuliert.

Ganz allgemein und nicht nur bezogen auf die hier beschriebenen Ausführungsbeispiele gilt Folgendes. Bei der akustischen Stimulation ist jeder Stimulationskanal einem jeweiligen unterschiedlichen Frequenzbereich zugeordnet, aus dem die Töne, die als akustische Reize in dem jeweiligen Stimulationskanal appliziert werden, ausgewählt werden. Bei der visuellen Stimulation werden die Stimulationskanäle durch unterschiedliche Stellen oder Bereiche im Gesichtsfeld des Patienten bestimmt. Die in einem jeweiligen Stimulationskanal erzeugten visuellen Reize werden an einer jeweiligen Stelle bzw. in einem jeweiligen Bereich des Gesichtsfelds erzeugt. Die Stimulationskanäle der taktilen, vibratorischen, thermischen, transkutanen elektrischen und/oder transkutanen magnetischen Reize und/oder Ultraschall-Reize werden durch die Stellen der Haut, welche mit den jeweiligen Stimulationselementen stimuliert werden, bestimmt. Folglich ist jeder Stimulationskanal einer jeweiligen Stelle bzw. einem jeweiligen Bereich der Haut zugeordnet.

Die Stimulationskanäle der gustatorischen Reize werden durch die Stellen der Zunge, welche mit den entsprechenden Geschmacksqualitäten oder elektrischen Reizen stimuliert werden, bestimmt. Bei einer olfaktorischen Stimulation verwendet man psychophysisch hinreichend disjunkte Geruchsreize, durch welche die Stimulationskanäle festgelegt würden. Die psychophysisch hinreichend disjunkten Geruchsreize könnten z. B. personalisiert, d. h. an den individuellen Patienten angepasst sein.

Bei der transkraniellen elektrischen und transkraniellen magnetischen Stimulation werden Elektroden bzw. Magnetfeldgeneratoren, insbesondere stromdurchflossene Spulen, am Körper, insbesondere am Kopf, des Patienten befestigt. Durch die Elektroden und Magnetfeldgeneratoren können Ströme bzw. Magnetfelder im Gehirn und/oder Rückenmark des Patienten erzeugt werden. Je nach Anbringungsort der Elektroden bzw. Magnetfeldgeneratoren können unterschiedliche Zielgebiete im Gehirn und/oder Rückenmark stimuliert werden. Die Stimulationskanäle werden folglich durch die Stellen am Körper des Patienten, an denen die Elektroden bzw. Magnetfeldgeneratoren angebracht sind, bestimmt.

Die vorstehend beschriebene Stimulationseinheit kann demnach unterschiedliche Bereiche des Gehirns oder Rückenmarks über verschiedene Stimulationskanäle separat stimulieren, indem die applizierten Reize über Nervenleitungen an unterschiedliche Zielgebiete, die im Gehirn und/oder Rückenmark liegen, weitergeleitet werden. Die Zielgebiete können während der Stimulation mit eventuell unterschiedlichen und/oder zeitversetzten Reizen stimuliert werden.

Wie oben beschrieben bewirken die Reize 22 bei der CR-Stimulation ein Zurücksetzen, einen sogenannten Reset, der Phase der neuronalen Aktivität der stimulierten Neuronen. Mit Hilfe der von der Messeinheit 16 aufgenommenen Messsignale 23 kann die Phasenrücksetzung der einzelnen Reize 22 überprüft werden. Eine derartige Untersuchung kann vor der eigentlichen therapeutischen Neurostimulation vorgenommen werden.

Dazu wird über einen Sensor der Messeinheit 16 ein Signal gemessen, welches die Aktivität der über den j-ten Stimulationskanal stimulierten Subpopulation hinreichend repräsentiert. Man erhält dieses Signal entweder direkt von der Subpopulation über eine nicht-invasive Messung, z. B. über EEG- oder MEG-Elektroden, oder eine invasive Messung, z. B. über implantierte Elektroden, als Oberflächen-EEG oder als lokales Feldpotential über Tiefenelektroden. Das Signal kann auch indirekt über die Messung einer mit der Aktivität der stimulierten Subpopulation korrelierten Größe ermittelt werden. Hierzu eignen sich z. B. EEG-/MEG-/LFP-Signale der neuronalen Aktivität einer mit dieser Subpopulation eng gekoppelten anderen Neuronenpopulation oder zugehörige Elektromyographie-, Akzelerometer- oder Gyroskop-Signale.

Da neuronale Signale typischerweise rhythmische Aktivität in unterschiedlichen Frequenzbändern enthalten, ist es in solchen Fällen vorteilhaft, z. B. mittels Bandpassfilterung oder wavelet-Analyse oder empirical mode decomposition das Signal $x_j(t)$, welches die pathologische oszillatorische Aktivität der vom j-ten Stimulationskanal stimulierten Subpopulation repräsentiert, zu ermitteln.

Ein nur wenig aufwändiges Vorgehen, um eine Phasenrücksetzung zu überprüfen, besteht darin, die gemittelte Reizantwort zu bestimmen. Hierzu wird zu den Zeiten $\tau_1$, $\tau_2$, ..., $\tau_l$ ein Reiz mit identischen Reizparametern appliziert. Die Abstände zwischen den einzelnen Reizen $\tau_{k+1} - \tau_k$ sollten hinreichend groß und randomisiert, also nicht konstant sein, um Einschwingvorgänge zu vermeiden (vgl. P. A. Tass: Transmission of stimulus-locked responses in two coupled phase oscillators. Phys. Rev. E 69, 051909-1-24 (2004)). Typischerweise sollten die Abstände $\tau_{k+1} - \tau_k$ im Bereich von mindestens dem Zehnfachen, besser dem Hundertfachen der mittleren Periode des pathologischen Oszillation liegen. Die über alle 1 Test-Reize gemittelte Reizantwort wird gemäß folgender Gleichung berechnet:

$$\overline{x}_j(t) = \frac{1}{l}\sum_{k=1}^{l} x_j(\tau_k + t)$$

$$(1)$$

Sofern die Abstände $\tau_{k+1} - \tau_k$ zwischen den einzelnen Reizen hinreichend groß sind, erhält man im prä-Stimulus-Bereich, d. h. im Bereich vor der Applikation eines jeweiligen Reizes, keine gemittelte Reizantwort (vgl. P. A. Tass: Transmission of stimulus-locked responses in two coupled phase oscillators. Phys. Rev. E 69, 051909-1-24 (2004)). Eine Phasenrücksetzung kann festgestellt werden, wenn eine gemittelte Reizantwort detektiert werden kann, d. h., wenn sich im post-Stimulus-Bereich, d. h. im Bereich für t > 0, wobei t = 0 den Anfangszeitpunkt des jeweiligen Reizes darstellt, eine von Null verschiedene Reizantwort findet. Dies kann durch visuelle Inspektion ermittelt werden. Man kann dies auch von der Vorrichtung 2, insbesondere der Steuereinheit 10, durchführen lassen, indem man die prä-Stimulus-Verteilung von $\overline{x}_j(t)$ oder $|\overline{x}_j(t)|$ betrachtet und einen charakteristischen Schwellwert, z. B. die 99te Perzentile der prä-Stimulus-Verteilung von $|\overline{x}_j(t)|$ oder schlicht deren Maximum bestimmt. Wenn nun z. B. der Betrag der post-Stimulus-Antwort prinzipiell oder für eine vorgegebene Mindestdauer, z. B. 20 ms, diesen charakteristischen Schwellenwert übersteigt, liegt eine von Null verschiedene gemittelte Antwort vor. In diesem Fall kann eine Phasenrücksetzung vorliegen. D. h., die Reizstärke müsste so lange erhöht werden, bis die post-Stimulus-Antwort sich von einer Nulllinie unterscheidet. Neben dem hier vorgestellten einfachen, aber in der Praxis bewährten Verfahren können auch andere, dem Fachmann bekannte statistische Tests zur Signalanalyse herangezogen werden.

Eine genauere, aber aufwändigere Variante zur Untersuchung, ob die Reize eine Phasenrücksetzung bewirken, bietet die Analyse der Phase.

Hierzu wird die Phase $\Psi_j(t)$ von $x_j(t)$ bestimmt. Dies erfolgt mittels Hilbert-Transformation aus dem mittels Bandpassfilterung bzw. empirical mode decomposition bestimmten Signal, welches die pathologische oszillatorische Aktivität repräsentiert. Die empirical mode decomposition ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454:903-995 (1998)). Die Kombination von empirical mode decomposition mit nachfolgender Hilbert-Transformation wird als Hilbert-Huang-Transformation bezeichnet (vgl. N. E. Huang et al.: A confidence limit for the empirical mode decomposition and Hilbert spectral analysis, Proceedings of the Royal Society of London Series A, 459, 2317-2345 (2003)). Die Phase $\Psi_j(t)$ kann auch mittels Wavelet-Analyse bestimmt werden.

Eine Phasenrücksetzung liegt vor, wenn die Phase $\Psi_j(t)$ durch einen Reiz (mit Reiz-Beginn bei t = 0) nach einer bestimmten Zeit auf einen Vorzugswert gesetzt wird. D. h., $\{\Psi_j(\tau_k + t)\}_{k=1,...,l}$, die von den 1 Reizantworten gewonnene Verteilung der Werte der Phase $\Psi_j(t)$ hat zur Zeit t (relativ zum Burst-Beginn bei t = 0) einen Häufungswert. Dem Fachmann sind unterschiedliche Methoden bekannt, mit denen sich nachweisen lässt, dass eine Verteilung einen Häufungswert (also einen Peak) hat. Eine gebräuchliche Methode ist die Bestimmung des Phasenrücksetzungsindex p(t) mittels zirkulärem Mittelwert:

$$\rho(t) = \left| \frac{1}{l} \sum_{k=1}^{l} \exp\left[ i \psi_j(\tau_k + t) \right] \right| \tag{2}$$

Eine Phasenrücksetzung liegt vor, wenn p(t) z. B. das Maximum oder die 99te Perzentile der prä-Stimulus-Verteilung von p(t) (an einem Zeitpunkt oder innerhalb eines kleinen Zeitfensters von z. B. 20 ms Breite) überschreitet.

In der Praxis hat sich die Analyse mit den gemittelten Antworten $\bar{x}_j(t)$ als ausreichend bewährt.

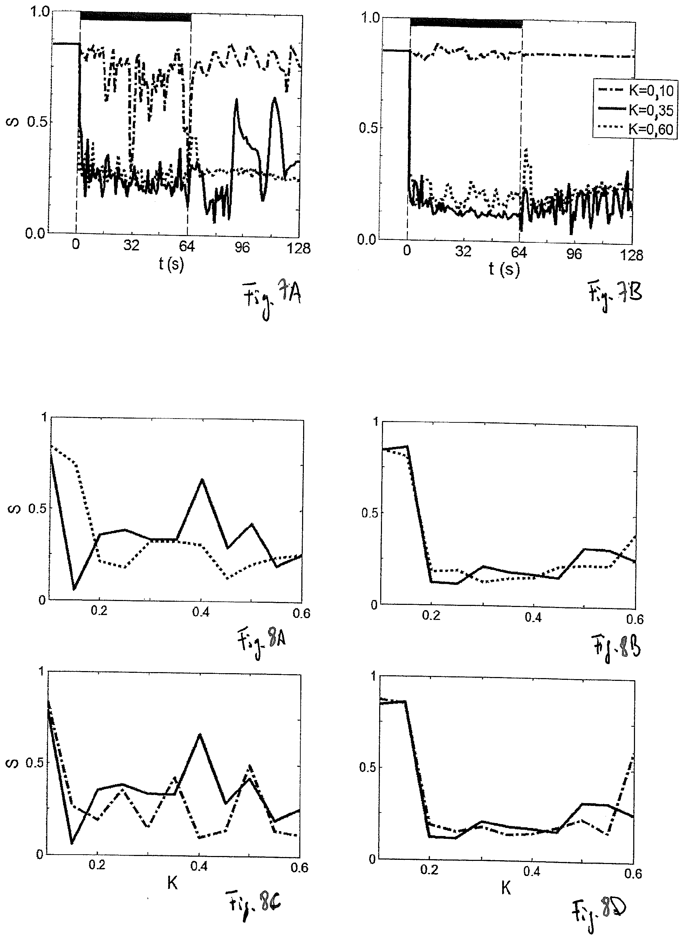

In den Fig. 7 bis 9 werden die mit der hierin beschriebenen Erfindung erzielbaren Effekte anhand von Simulationsergebnissen veranschaulicht. Der Simulation liegt ein Netzwerk von 200 Neuronen zugrunde, wobei alle Neuronen untereinander eine starke anregende kurzreichweitige Kopplung und eine schwache inhibitorische langreichweitige Kopplung aufweisen. Die synaptischen Kopplungsstärken in dem Netzwerk können sich gemäß STDP (Spike Timing-Dependent Plasticity)-Regeln ändern. Ein anfänglich stark gekoppeltes Netzwerk produziert hoch synchrone neuronale Aktivität.

Die Simulation basiert auf folgenden Randbedingungen. Die CR-Stimulation startet bei t = 0 s und endet bei t = 64 s. Jeder Zyklus dauert 16 ms. Ein Muster aus 3 Zyklen mit Stimulation und 2 Zyklen ohne Stimulation wird periodisch wiederholt. Die Aktivität des Netzwerks wird bis t = 128 s untersucht, d. h., bis 64 s nach Ende der Stimulation. Der Synchronisationsgrad S kann im Bereich von 0 (für eine komplette Desynchronisation) bis 1 (für eine vollständige Phasensynchronisation) liegen.

In den Fig. 7A und 7B ist der Synchronisationsgrad S der simulierten Neuronenpopulation mit einer krankhaft synchronen und oszillatorischen neuronalen Aktivität vor, während und nach einer CR-Stimulation dargestellt. Die in den beiden Darstellungen oben eingezeichneten horizontalen Balken geben den Zeitraum an, in dem die CR-Stimulation angewandt wird. Bei der in Fig. 7A dargestellten Simulation wurden die Sequenzen zu Beginn jedes Zyklus variiert, während die Sequenzen bei der in Fig. 7B dargestellten Simulation erst nach jeweils 100 Zyklen variiert wurden. Der Synchronisationsgrad S wurde nach jeder Millisekunde berechnet. In den Fig. 7A und 7B ist jeweils die 50. Perzentile der Werte für den Synchronisationsgrad S für drei verschiedene Stimulationsstärken K dargestellt.

Als Ergebnis lässt sich den Fig. 7A und 7B entnehmen, dass bis auf die CR-Stimulation mit einer Stimulationsstärke K von 0,10 die in Fig. 7B dargestellte langsam variierende CR-Stimulation einen größeren und auch länger anhaltenden Stimulationserfolg zeigt.

Neben der Stimulationsstärke K haben auch die Reihenfolge der Sequenzen und die Anfangsbedingungen des Netzwerks Einfluss auf den Stimulationserfolg. Dies ist in den Fig. 8A bis 8D gezeigt, in denen der Synchronisationsgrad S gegen die Stimulationsstärke K aufgetragen ist. Den in den Fig. 8A und 8C dargestellten Simulationen liegt eine Variation der Sequenzen in jedem Zyklus zugrunde, wohingegen bei den in den Fig. 8B und 8D gezeigten Simulationen die Sequenzen erst nach 100 Zyklen variiert wurden.

In den Fig. 8A und 8B ist jeweils die 50. Perzentile der Werte für den Synchronisationsgrad S für die letzten 16 Sekunden der Stimulation als eine Funktion der Stimulationsstärke K für zwei verschiedene Reihenfolgen der Sequenzen dargestellt. Die durchgezogenen Linien zeigen die Simulationsergebnisse für die gleiche Reihenfolge der Sequenzen, wie sie auch den Simulationen von Fig. 7A und 7B zugrunde lag, während die punktierten Linien die Simulationsergebnisse für eine andere Reihenfolge darstellen. Die Fig. 8A und 8B zeigen, dass die langsam variierende CR-Stimulation im Vergleich zur schnell variierenden CR-Stimulation robuster gegenüber der Reihenfolge der Sequenzen ist.

Die Simulationen zeigen darüber hinaus, dass auch die Anfangsbedingungen des Netzwerks Einfluss auf die Desynchronisation der neuronalen Aktivität haben. In den Fig. 8C und 8D ist jeweils die 50. Perzentile der Werte für den Synchronisationsgrad S für die letzten 16 Sekunden der Stimulation als eine Funktion der Stimulationsstärke K für zwei verschiedene Anfangsbedingungen des Netzwerks dargestellt. Die durchgezogenen Linien zeigen die Simulationsergebnisse für die gleichen Anfangsbedingungen des Netzwerks, wie sie auch den Simulationen von Fig. 7A und 7B zugrunde lagen, während die strichpunktierten Linien die Simulationsergebnisse für andere Anfangsbedingungen des Netzwerks darstellen. Auch hier zeigt sich die langsam variierende CR-Stimulation robuster gegenüber den Anfangsbedingungen des Netzwerks im Vergleich zur schnell variierenden CR-Stimulation.

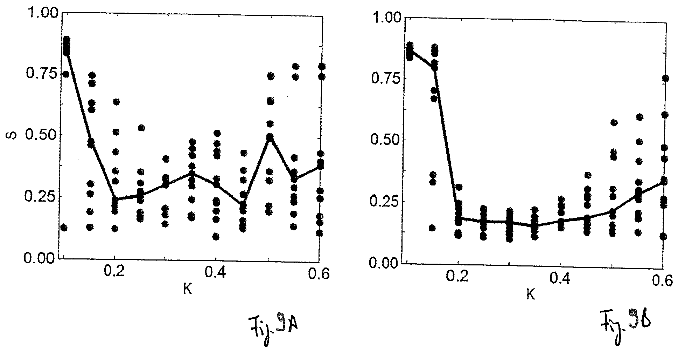

Sowohl für die schnell variierende als auch die langsam variierende CR-Stimulation wurden 10 weitere Simulationen mit unterschiedlichen Reihenfolgen der Sequenzen sowie unterschiedlichen Anfangsbedingungen des Netzwerks durchgeführt. Die Ergebnisse dieser Simulationen sind in den Fig. 9A und 9B gezeigt, in denen die 50. Perzentile der Werte für den Synchronisationsgrad S für die letzten 16 Sekunden der Stimulation als eine Funktion der Stimulationsstärke K für verschiedene Reihenfolgen der Sequenzen und verschiedene Anfangsbedingungen der Netzwerks aufgetragen ist. Der Median der Ergebnisse für jeden Wert der Stimulationsstärke K ist jeweils durch eine Linie verbunden. Fig. 9A zeigt die Ergebnisse der schnell variierenden CR-Stimulation, und Fig. 9B zeigt die Ergebnisse der langsam variierenden CR-Stimulation. Als Ergebnis lässt sich den Fig. 9A und 9B entnehmen, dass die langsam variierende CR-Stimulation das hoch synchrone neuronale Netzwerk stärker und robuster desynchronisiert, als dies mit der schnell variierenden CR-Stimulation möglich ist.

## Patentansprüche

1. Vorrichtung (1; 2) zur Stimulation von Neuronen (31), umfassend

   - eine nicht-invasive Stimulationseinheit (11) zur Erzeugung von Reizen (22) in einer Mehrzahl von Stimulationskanälen (12-15), wobei die Stimulationseinheit (11) derart ausgelegt ist, dass die Reize (22) eine Neuronenpopulation (31) im Gehirn und/oder Rückenmark (30) eines Patienten über die Stimulationskanäle (12-15) an jeweils unterschiedlichen Stellen stimulieren, und
   - eine Steuereinheit (10), welche die Stimulationseinheit (11) derart ansteuert, dass die Stimulationseinheit (11) repetitiv Sequenzen von Reizen (22) erzeugt, wobei
   - die Reihenfolge der Stimulationskanäle (12-15), in denen die Reize (22) innerhalb einer Sequenz erzeugt werden, für mindestens 20 nacheinander generierte Sequenzen konstant ist und danach variiert wird.

2. Vorrichtung (1; 2) nach Anspruch 1, wobei die Reize (22) akustische, visuelle, taktile, vibratorische, thermische, olfaktorische, gustatorische, transkutane elektrische, transkutane magnetische, transkranielle elektrische und/oder transkranielle magnetische Reize und/oder Ultraschall-Reize sind.

3. Vorrichtung (1; 2) nach Anspruch 1 oder 2, wobei die Sequenzen in einem Zeitraster, das aus aufeinanderfolgenden Zyklen besteht, erzeugt werden und zumindest in einigen der Zyklen jeweils eine Sequenz von Reizen (22) erzeugt wird.

4. Vorrichtung (1; 2) nach Anspruch 3, wobei innerhalb eines jeweiligen Zyklus entweder genau eine Sequenz von Reizen (22) erzeugt wird oder keine Reize erzeugt werden.

5. Vorrichtung (1; 2) nach Anspruch 3 oder 4, wobei während n aufeinanderfolgenden Zyklen Reize (22) erzeugt werden und während der darauffolgenden m Zyklen keine Reize erzeugt werden und dieses Muster periodisch fortgesetzt wird, wobei n und m nicht-negative ganze Zahlen sind.

6. Vorrichtung (1; 2) nach einem der vorhergehenden Ansprüche, wobei das Muster, nach welchem die Reihenfolge der Stimulationskanäle (12-15), in denen die Reize (22) innerhalb einer Sequenz erzeugt werden, für mindestens 20 nacheinander generierte Sequenzen konstant ist und danach variiert wird, mehrfach wiederholt wird.

7. Vorrichtung (1; 2) nach einem der vorhergehenden Ansprüche, wobei die Reize (22) dazu ausgelegt sind, bei einer Verabreichung an den Patienten über die Mehrzahl von Stimulationskanälen (12-15) eine krankhaft synchrone und oszillatorische Aktivität der Neuronenpopulation (31) zu unterdrücken.

8. Vorrichtung (1; 2) nach Anspruch 7, wobei die Dauer eines Zyklus im Wesentlichen der mittleren Periode der pathologischen Oszillation der Neuronen (31) entspricht.

9. Vorrichtung (1; 2) nach einem der vorhergehenden Ansprüche, wobei die Stimulationseinheit (11) derart ausgelegt ist, dass die in einem jeweiligen Stimulationskanal (12-15) generierten Reize (22) eine jeweilige Subpopulation (32-35) der Neuronenpopulation (31) stimulieren und die Phase der neuronalen Aktivität dieser Subpopulation (32-35) zurücksetzen.

10. Vorrichtung (1; 2) nach einem der vorhergehenden Ansprüche, wobei die Stimulationseinheit (11) innerhalb einer jeweiligen Sequenz in jedem Stimulationskanal (11-15) genau einen Reiz (22) erzeugt.

11. Vorrichtung (1; 2) nach Anspruch 10, wobei genau ein Reiz (22) genau ein Pulszug ist.

12. Vorrichtung (2) nach einem der vorhergehenden Ansprüche, umfassend eine Messeinheit (16) zum Aufnehmen von Messsignalen (23), die eine neuronale Aktivität der mit den Reizen (22) stimulierten Neuronenpopulation (31) wiedergeben.

13. Vorrichtung (2) nach Anspruch 12, wobei die Steuereinheit (10) derart ausgelegt ist, dass sie die Anzahl der nacheinander generierten Sequenzen, in denen die Reihenfolge der Stimulationskanäle (12-15), in denen die Reize (22) innerhalb einer Sequenz erzeugt werden, konstant ist, verlängert, wenn die Steuereinheit (10) anhand der Messsignale (23) feststellt, dass ein Synchronisationsgrad der stimulierten Neuronenpopulation (31) durch die Applikation der Reize (22) nicht um mindestens einen vorgegebenen Schwellwert reduziert wird.

14. Ein Computerprogramm, das aus einem Software-Code besteht, der ausgebildet ist, eine Vorrichtung nach Anspruch 1 zu veranlassen,

- Steuersignale zum Ansteuern einer nicht-invasiven Stimulationseinheit (11) zu erzeugen, wobei die Stimulationseinheit (11) Reize (22) in einer Mehrzahl von Stimulationskanälen (12-15) erzeugt und die Reize (22) eine Neuronenpopulation (31) im Gehirn und/oder Rückenmark (30) eines Patienten über die Stimulationskanäle (12-15) an jeweils unterschiedlichen Stellen stimulieren, wobei die Steuersignale die Stimulationseinheit (11) derart ansteuern, dass
- die Stimulationseinheit (11) repetitiv Sequenzen von Reizen (22) erzeugt, und
- die Reihenfolge der Stimulationskanäle (12-15), in denen die Reize (22) innerhalb einer Sequenz erzeugt werden, für mindestens 20 nacheinander generierte Sequenzen konstant ist und danach variiert wird.

**Claims**

1. An apparatus (1; 2) for stimulating neurons (31), the apparatus comprising:

a non-invasive stimulation unit (11) configured to generate stimuli (22) in a plurality of stimulation channels (12-15), wherein the stimulation unit (11) is configured such that the stimuli (22) stimulate a neuronal population (31) in the brain and/or the spinal cord (30) of a patient over the stimulation channels (12-15) at respective different points; and
a control unit (10) configured to control the stimulation unit (11) to generate repetitive sequences of the stimuli (22),
wherein an order of the plurality of stimulation channels (12-15) in which the stimuli (22) are generated within each sequence is kept constant for at least 20 sequences generated after one another and is varied thereafter.

2. The apparatus (1; 2) in accordance with claim 1, wherein the stimuli (22) are acoustic stimuli, visual stimuli, tactile stimuli, vibratory stimuli, thermal stimuli, olfactory stimuli, gustatory stimuli, transcutaneous electrical stimuli, transcutaneous magnetic stimuli, transcranial electrical stimuli and/or transcranial magnetic stimuli and/or ultrasound stimuli.

3. The apparatus (1; 2) in accordance with claim 1 or 2, wherein the sequences are generated in a time pattern that consists of consecutive cycles, and wherein at least one respective sequence of stimuli (22) is generated in at least some of the consecutive cycles.

4. The apparatus (1; 2) in accordance with claim 3, wherein either exactly one sequence of stimuli (22) is generated or no stimuli are generated within a respective cycle.

5. The apparatus (1; 2) in accordance with claim 3 or 4, wherein stimuli (22) are generated during n consecutive cycles and no stimuli are generated during m cycles following the n consecutive cycles, and this pattern is periodically continued, wherein n and m are non-negative integers.

6. The apparatus (1; 2) in accordance with any one of the preceding claims, wherein the pattern according to which the order of the stimulation channels (12-15) in which the stimuli are generated within a sequence is kept constant for at least 20 sequences generated after one another and is varied thereafter is repeated a plurality of times.

7. The apparatus (1; 2) in accordance with any one of the preceding claims, wherein the stimuli (22) are configured to suppress a pathologically synchronous and oscillatory activity of the neuron population (31) upon administration to the patient over the plurality of stimulation channels (12 -15).

8. The apparatus (1; 2) in accordance with claim 7, wherein a duration of a cycle substantially corresponds to the mean period of the pathological oscillation of the neurons (31).

9. The apparatus (1; 2) in accordance with any one of the preceding claims, wherein the stimulation unit (11) is configured such that the stimuli (22) generated in a respective stimulation channel (12-15) stimulate a respective subpopulation (32-35) of the neuronal population (31) and reset the phase of the neuronal activity of this subpopulation (32-35).

10. The apparatus (1; 2) in accordance with any one of the preceding claims, wherein the stimulation unit (11) generates exactly one stimulus (22) within a respective sequence in each stimulation channel (12-15).

11. The apparatus (1; 2) in accordance with claim 10, wherein the exactly one stimulus (22) is exactly one pulse train.

12. The apparatus (1; 2) in accordance with any one of the preceding claims, further comprising a measuring unit (16) configured to record measured signals (23) that reproduce a neuronal activity of the neuronal population (31) stimulated by the stimuli (22).

13. The apparatus (1; 2) in accordance with claim 12, wherein the control unit (10) is further configured to extend a number of the consecutively generated sequences in which the order of the stimulation channels (12-15) in which the stimuli are generated within a sequence is constant when the control unit (10) determines, based on the measured signals (23), that a degree of synchronization of the stimulated neuron population (31) is not reduced by at least one predefined threshold value by application of the stimuli (22).

14. A computer program consisting of software code that is configured to cause an apparatus in accordance with claim 1:

    to generate control signals to control a non-invasive stimulation unit (11), wherein the stimulation unit (11) generates stimuli (22) in a plurality of stimulation channels (12-15) and the stimuli stimulate a neuronal population (31) in the brain and/or the spinal cord (30) of a patient over the stimulation channels (12-15) at respective different points;
    wherein the control signals control the stimulation unit (11) such that:

        the stimulation unit (11) generates repetitive sequences of the stimuli (22) and
        an order of the stimulation channels (12-15) in which the stimuli (22) are generated within a sequence is constant for at least 20 sequences generated after one another and is varied thereafter.

**Revendications**

1. Dispositif (1 ; 2) pour stimuler des neurones (31) comprenant

    - une unité de stimulation non-invasive (11) pour générer des stimuli (22) dans une pluralité de canaux de stimulation (12-15), sachant que l'unité de stimulation (11) est conçue de manière que les stimuli (22) stimulent une population de neurones (31) dans le cerveau et/ou dans la moelle épinière (30) d'un patient à différents emplacements respectifs via les canaux de stimulation (12-15), et
- une unité de commande (10) qui commande l'unité de stimulation (11) de telle manière que l'unité de stimulation (11) produise des séquences répétitives de stimuli (22), sachant que
- l'ordre des canaux de stimulation (12-15) dans lesquels les stimuli (22) sont générés à l'intérieur d'une séquence est constant pour au moins 20 séquences générées successivement, et qu'il est ensuite varié.

2. Dispositif (1 ; 2) d'après la revendication 1, sachant que les stimuli (22) sont des stimuli acoustiques, visuels, tactiles, vibratoires, thermiques, olfactifs, gustatifs, transcutanés électriques, transcutanés magnétiques, transcrâniens électriques et/ou transcrâniens magnétiques et/ou ultrasoniques.

3. Dispositif (1 ; 2) d'après la revendication 1 ou 2, sachant que les séquences sont générées dans une grille de temps constituée de cycles successifs et qu'au moins dans certains des cycles est générée respectivement une séquence de stimuli (22).

4. Dispositif (1 ; 2) d'après la revendication 3, sachant que, dans un cycle respectif, soit exactement une séquence de stimuli (22) est générée, soit aucun stimuli n'est généré.

5. Dispositif (1 ; 2) d'après la revendication 3 ou 4, sachant que des stimuli (22) sont générés pendant **n** cycles consécutifs et aucun stimulus n'est généré pendant les **m** cycles suivants et que ce modèle est poursuivi périodiquement, sachant que **n** et **m** sont des nombres entiers non négatifs.

6. Dispositif (1 ; 2) d'après une quelconque des revendications précédentes, sachant que le modèle, d'après lequel la séquence de canaux de stimulation (12-15), dans laquelle les stimuli (22) sont générés à l'intérieur d'une séquence, est constante pendant au moins 20 séquences générées successivement et est ensuite modifiée, est répété plusieurs fois.

7. Dispositif (1 ; 2) d'après une quelconque des revendications précédentes, sachant que stimuli (22) sont conçus pour supprimer l'activité pathologiquement synchrone et oscillatoire de la population de neurones (31) lorsqu'ils sont administrés au patient par l'intermédiaire de la pluralité de canaux de stimulation (12-15).

8. Dispositif (1 ; 2) d'après la revendication 7, sachant que la durée d'un cycle correspond essentiellement à la période moyenne de l'oscillation pathologique des neurones (31).

9. Dispositif (1 ; 2) d'après une quelconque des revendications précédentes, sachant que l'unité de stimulation (11) est conçue de telle manière que les stimuli (22) générés dans un canal de stimulation (12-15) respectif stimulent une sous-population (32-35) respective de la population neuronale (31) et réinitialisent la phase de l'activité neuronale de cette sous-population (32-35).

10. Dispositif (1 ; 2) d'après une quelconque des revendications précédentes, sachant que l'unité de stimulation (11) génère à l'intérieur d'une séquence respective exactement un stimulus (22) dans chaque canal de stimulation (11-15).

11. Dispositif (1 ; 2) d'après la revendication 10, sachant que exactement un stimulus (22) est exactement un train d'impulsions.

12. Dispositif (2) d'après une quelconque des revendications précédentes, comprenant une unité de mesure (16) pour recevoir ou encore enregistrer des signaux de mesure (23) représentant une activité neuronale de la population de neurones (31) stimulée par les stimuli (22).

13. Dispositif (2) d'après la revendication 12, sachant que l'unité de commande (10) est conçue de telle manière qu'elle étend le nombre de séquences générées successivement dans lesquelles la séquence de canaux de stimulation (12-15), dans laquelle les stimuli (22) sont générés dans une séquence, est constante, lorsque ladite unité de commande (10) détermine à partir des signaux de mesure (23) qu'un degré de synchronisation de la population de neurones stimulée (31) n'est pas réduit d'au moins une valeur seuil prédéterminée par l'application des stimuli (22).

14. Programme informatique consistant en un code logiciel conçu pour amener un dispositif d'après la revendication 1 à

- générer des signaux de commande pour la commande d'une unité de stimulation non-invasive (11), sachant que l'unité de stimulation (11) génère des stimuli (22) dans une pluralité de canaux de stimulation (12-15) et que les stimuli (22) stimulent une population de neurones (31) dans le cerveau et/ou dans la moelle épinière (30) d'un patient via les canaux de stimulation (12-15) à différents emplacements respectifs, les signaux de commande entraînant l'unité de stimulation (11) de manière que

- l'unité de stimulation (11) génère de manière répétitive des séquences de stimuli (22), et que
- l'ordre des canaux de stimulation (12-15), dans lesquels les stimuli (22) sont générés à l'intérieur d'une séquence, est constant pour au moins 20 séquences générées successivement et qu'il est ensuite varié.

Fig. 1

Fig. 2A

Fig. 2B

Fig. 3

Fig. 4

Fig. 5

Fig. 6

Fig. 7A

Fig. 7B

K=0,10
K=0,35
K=0,60

Fig. 8A

Fig. 8B

Fig. 8C

Fig. 8D

Fig. 9A

Fig. 9B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010000390 A

**In der Beschreibung aufgeführte Nicht-Patentliteratur**

- **P. A. TASS ; I. ADAMCHIC ; H.-J. FREUND ; T. VON STACKELBERG ; C. HAUPTMANN.** Counteracting tinnitus by acoustic coordinated reset neuromodulation. *Restorative Neurology and Neuroscience,* 2012, vol. 30, 137-159
- **P. A. TASS ; I. ADAMCHIC ; H.-J. FREUND ; T. VON STACKELBERG ; C.HAUPTMANN.** Counteracting tinnitus by acoustic coordinated reset neuromodulation. *Restorative Neurology and Neuroscience,* 2012, vol. 30, 137-159
- **I. ADAMCHIC ; T. TOTH ; C. HAUPTMANN ; P. A. TASS.** Reversing pathologically increased EEG power by acoustic CR neuromodulation. *Human Brain Mapping,* 2014, vol. 35, 2099-2118
- **A. N. SILCHENKO ; I. ADAMCHIC ; C. HAUPTMANN ; P. A. TASS.** Impact of acoustic coordinated reset neuromodulation on effective connectivity in a neural network of phantom sound. *Neuroimage,* 2013, vol. 77, 133-147
- **I. ADAMCHIC ; B. LANGGUTH ; C. HAUPTMANN ; P. A. TASS.** Abnormal brain activity and cross-frequency coupling in the tinnitus network. *Frontiers in Neuroscience,* 2014, vol. 8, 284
- **P. A. TASS.** Transmission of stimulus-locked responses in two coupled phase oscillators. *Phys. Rev. E,* 2004, vol. 69, 051909-1, 24
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345